# EUROPEAN PATENT APPLICATION

(11) **EP 4 847 911 A1**
(43) Date of publication of application: **02.12.2026**
(21) Application number: 26164135.1
(22) Date of filing: 11.03.2026
(51) Int. Cl.: G01N 23/223, G01N 33/202

(54) **AUTOMATED METAL ALLOY ANALYSIS USING HAND-HELD APPARATUS**

(30) Priority: 30.05.2025 US 202563814699 P; 24.06.2025 NL 2040626
(71) Applicant: The Boeing Company, Arlington, VA 22202 (US)
(72) Inventor: NWOKE, Dominic Ndubueze, Arlington, 22202 (US); HENSHAW, Dana Alexander, Arlington, 22202 (US)
(74) Representative: Arnold & Siedsma

(57) **Abstract**

Techniques for evaluating a metal alloy sample are presented. The techniques include: obtaining a chemical composition quantification for the metal alloy sample, where the chemical composition quantification is obtained from spectroscopic data provided by a portable hand-held X-ray fluorescence spectrometer; passing, automatically, the chemical composition quantification to a trained machine learning system, from which a quantitative source estimation is obtained, where the trained machine learning system is trained with a chemical composition quantification training corpus, where the chemical composition quantification training corpus includes a plurality of training chemical composition quantifications characterizing training metal alloy samples that originate from a plurality of metal alloy sources; and providing a source evaluation of the metal alloy sample, where the source evaluation of the metal alloy sample is based on the quantitative source estimation, and where the source evaluation of the metal alloy sample includes a particular metal alloy source.

## Description

### Field

This disclosure relates generally to chemical analysis of materials such as metal alloys.

### Background

Metal alloys are used in many applications in various contexts, including in the biomedical, automotive, and aerospace industries. For some applications, a manufacturer may certify one or more metal alloy sources for providing a particular metal alloy. The certification process may include the manufacturer specifying a required chemical composition of the particular metal alloy (e.g., in terms of constituent elements), as well as one or more additional requirements regarding non-chemical-composition properties, such as tensile strength (e.g., in terms of psi, N/M2, or MPa), ductile quantification (e.g., in terms of elongation percentage), microstructure (e.g., with respect to the presence and/or ratio of certain crystalline phases, such as alpha and beta), and others.

A metal alloy source that wishes to be certified may determine an internal alloying process for creating the particular metal alloy. The metal alloy source may present samples of the particular metal alloy created using its alloying process to the manufacturer. The manufacturer may conduct extensive, expensive, and time-consuming tests of the samples in order to determine whether they comply with the chemical composition and other non-chemical-composition requirements. Such testing may include any, or a combination, of: quantitative chemical analysis by chemical titration, automated fatigue testing, tensile testing, ductility testing, X ray crystallography, electron microscopy, etc. If the provided samples comply, the manufacturer may certify the metal alloy source for supplying the particular metal alloy.

Part of the certification may include the metal alloy source agreeing to always use the same metal alloying process to produce the particular metal alloy. Use of the same metal alloying process facilitates production of generally homogenous metal alloy batches. The agreement to use the same alloying process gives the manufacturer confidence that the particular metal alloy supplied by a certified metal alloy source will not only have the required chemical composition, but also meet any other requirements regarding non-chemical-composition properties. This frees the manufacturer from having to subject each new batch of the particular metal alloy provided by a certified supplier to extensive, expensive, and time-consuming testing.

Nevertheless, batches of metal alloys that are indicated as originating from a given certified supplier, but that are actually produced by a different entity, may occasionally enter the supply chain. Such batches may escape detection by existing testing techniques due to small compositional differences that still remain within the specification limits of the required chemical composition. However, while they may technically fall within specification limits for elemental composition, the batches may not have been produced by the indicated certified supplier using its agreed-upon alloying process. Such batches may therefore fail to comply with one or more other requirements regarding non-chemical-composition properties, such as tensile strength, ductility, microstructure, etc. Of further concern are batches of alloys specified as being aerospace grade, but may not, in fact, have the requirements of being aerospace grade.

US 2013/299591 A1, according to its abstract, states encoding information in a material using a material tracing system which includes storing information to be encoded in the material, generating a number based on the information, determining an amount of at least one tracer to be incorporated into the material corresponding to the number, and incorporating the determined amount of the at least one tracer into the material. Decoding information encoded in the material includes measuring an amount of the at least one tracer, in some examples after tracer activation, determining a number corresponding to the measured at least one tracer, and decoding the number to obtain information associated with the material. A material tracing system is also said to be provided.

US 2013/230138 A1, according to its abstract, states a method and apparatus for the metallurgical qualitative analysis (assay) and valuation of precious metals objects such as jewelry or coins. The system integrates a commercially available x-ray florescence (XRF) metals analyzer with associated peripheral devices including a personal computer and keyboard or touchpad computing device, a digitizing scale, a printer, an Internet link for obtaining current precious metals market price quotations, and software for processing the qualitative results with the current market price data and presenting the results to the system operator in real time. The system optionally includes a customer interface including a display screen for presenting the customer with the results of the analysis and valuation, and customer input means for accepting and recording a purchase transaction.

US 2016/033430 A1, according to its abstract, states a handheld x-ray fluorescence (XRF) device that may include communication circuitry for communicating over a network. In some examples information related to sale and/or pricing of material identified by the XRF device is communicated by and/or to the XRF device. In some examples the XRF device communicates information regarding rental of the XRF device. In some examples the XRF device may be used to ascertain whether an item, for example an item of evidence or a particular item in a manufacturing environment, includes a material composition expected for the item, and in some examples storing or logging a result of such a determination.

CN 115 512 144 A, according to its abstract, states an XRF spectrogram automatic classification method based on a convolution auto-encoder, belonging to the field of XRF spectrum analysis processing. According to the method, normalization processing is carried out on the obtained XRF spectrum data to be detected, and the one-dimensional spectrum data vector is converted into the two-dimensional spectrum information matrix form after normalization processing, so that the XRF spectrum data can be processed by adopting a picture processing mode, and the classification precision is improved. On this basis, feature compression is carried out on the XRF spectrum to be measured by building a convolutional auto-encoder neural network model, and a spectrum data spectrum after feature compression is obtained, by designing the kmeans classification network, unsupervised classification is realized, and the problem of low classification precision and efficiency caused by complicated spectrogram characteristic indexes of the XRF spectrum is addressed.

The disclosure addresses the issue of obtaining rapid, accurate and consistent elemental analysis of alloys, and in embodiments, titanium alloys during manufacturing processing, and in embodiments, during high variability environments such as those found in aerospace manufacturing.

### Summary

According to various embodiments, a method, such as a computer-implemented method, of evaluating a metal alloy sample is presented. The method includes: obtaining a chemical composition quantification for the metal alloy sample, wherein the chemical composition quantification is obtained from spectroscopic data provided by a portable hand-held X-ray fluorescence spectrometer applied to the metal alloy sample; passing, automatically, the chemical composition quantification to a trained machine learning system, from which a quantitative source estimation is obtained, wherein the trained machine learning system is trained with a chemical composition quantification training corpus, wherein the chemical composition quantification training corpus comprises a plurality of training chemical composition quantifications characterizing training metal alloy samples that originate from a plurality of metal alloy sources; and providing a source evaluation of the metal alloy sample, wherein the source evaluation of the metal alloy sample is based on the quantitative source estimation, and wherein the source evaluation of the metal alloy sample comprises a particular metal alloy source.

Various optional features of the above method embodiments include the following. The chemical composition quantification training corpus may comprise the plurality of training chemical composition quantifications for training metal alloy samples that originate from one or more metal alloy sources, which may thereby characterize a metal alloy source fingerprint for each of the one or more metal alloy sources, the metal alloy source fingerprint representing an inherent chemical fingerprint. The metal alloy source fingerprint may represent an inherent chemical fingerprint. The portable hand-held X-ray fluorescence spectrometer may include the trained machine learning system. The providing may include providing on a display integrated into the portable hand-held X-ray fluorescence spectrometer. The trained machine learning system may be communicatively coupled to the portable hand-held X-ray fluorescence spectrometer, and the passing may comprise automatically wirelessly transmitting. The source evaluation of the metal alloy sample may include one of a binary value, wherein the binary value corresponds to whether or not the metal alloy sample originated from the particular metal alloy source and was produced by a metal alloying process certified for the particular metal alloy source. The source evaluation of the metal alloy sample may include a score, wherein the score characterizes a likelihood of whether the metal alloy sample originated from the particular metal alloy source and was produced by a metal alloying process certified for the particular metal alloy source. The quantitative source estimation may match a metal alloy source fingerprint that represents a particular metal alloying process of the particular metal alloy source. The trained machine learning system may be trained using a clustering algorithm, wherein the quantitative source estimation may comprise an identification of a closest cluster, and the source evaluation of the metal alloy sample may comprise a score representative of a likelihood that the metal alloy sample belongs to the closest cluster. The method may further include retraining the trained machine learning system with a chemical composition quantification retraining corpus, and the chemical composition quantification retraining corpus may comprise the chemical composition quantification. The source evaluation of the metal sample may include a plurality of scores representative of likelihoods that the metal alloy sample belongs to each of a plurality of previously identified clusters.

According to various embodiments, a system for evaluating a metal alloy sample is presented. The system includes: a portable hand-held X-ray fluorescence spectrometer configured to obtain spectroscopic data when applied to the metal alloy sample; a computer-readable medium, such as a non-transitory computer readable medium, comprising instructions; and at least one processor (e.g., electronic processor) that executes the instructions to perform operations comprising: obtaining a chemical composition quantification for the metal alloy sample, wherein the chemical composition quantification is obtained from the spectroscopic data; passing, automatically, the chemical composition quantification to a trained machine learning system, from which a quantitative source estimation is obtained, wherein the trained machine learning system is trained with a chemical composition quantification training corpus, wherein the chemical composition quantification training corpus comprises a plurality of training chemical composition quantifications characterizing training metal alloy samples that originate from a plurality of metal alloy sources; and providing a source evaluation of the metal alloy sample, wherein the source evaluation of the metal alloy sample is based on the quantitative source estimation, and wherein the source evaluation of the metal alloy sample comprises a particular metal alloy source.

Various optional features of the above system embodiments include the following. The chemical composition quantification training corpus may comprise the plurality of training chemical composition quantifications for training metal alloy samples that originate from one or more metal alloy sources, which may thereby characterize a metal alloy source fingerprint for each of the one or more metal alloy sources, the metal alloy source fingerprint representing an inherent chemical fingerprint. The metal alloy source fingerprint may represent an inherent chemical fingerprint. The portable hand-held X-ray fluorescence spectrometer may include the trained machine learning system. The portable hand-held X-ray fluorescence spectrometer may include an integrated display, and the providing may include providing on the display. The trained machine learning system may be communicatively coupled to the portable hand-held X-ray fluorescence spectrometer, and wherein the passing comprises automatically wirelessly transmitting. The source evaluation of the metal alloy sample may include one of a binary value, wherein the binary value corresponds to whether or not the metal alloy sample originated from the particular metal alloy source and was produced by a metal alloying process certified for the particular metal alloy source. The source evaluation of the metal alloy sample may include a score, wherein the score characterizes a likelihood of whether the metal alloy sample originated from the particular metal alloy source and was produced by a metal alloying process certified for the particular metal alloy source. The quantitative source estimation may match a metal alloy source fingerprint that represents a particular metal alloying process of the particular metal alloy source. The trained machine learning system may be trained using a clustering algorithm, wherein the quantitative source estimation may comprise an identification of a closest cluster, and the source evaluation of the metal alloy sample may comprise a score representative of a likelihood that the metal alloy sample belongs to the closest cluster. The operations may further include retraining the trained machine learning system with a chemical composition quantification retraining corpus, wherein the chemical composition quantification retraining corpus comprises the chemical composition quantification. The source evaluation of the metal sample may include a plurality of scores representative of likelihoods that the metal alloy sample belongs to each of a plurality of previously identified clusters.

Combinations, (including multiple dependent combinations) of the above-described elements and those within the specification have been contemplated by the inventors and may be made, except where otherwise indicated or where contradictory.

### Brief Description of the Drawings

Various features of the examples can be more fully appreciated, as the same become better understood with reference to the following detailed description of the examples when considered in connection with the accompanying figures, in which:
Fig. 1 is a schematic diagram of a system for evaluating a metal alloy sample, according to various embodiments;
Fig. 2 depicts clusters of chemical composition quantifications projected into a reduced dimensional space, according to various embodiments;
Fig. 3 depicts a non-limiting example chemical composition quantification, shown pictorially for purposes of illustration over a periodic table, according to various embodiments;
Fig. 4 depicts a portion of a chemical composition quantification, shown pictorially for purposes of illustration as a bar chart, according to various embodiments;
Fig. 5 depicts a portion of raw chemical composition data in domain of X-ray fluorescence and in the forms of a spectrogram line graph and a spectrogram color intensity graph, according to various embodiments;
Fig. 6 is a flow chart for a method of training and using a machine learning system for evaluating a metal alloy sample, according to various embodiments; and
Fig. 7 is a flow diagram for a method of evaluating a metal alloy sample, according to various embodiments.

### Description of the Examples

Reference will now be made in detail to example implementations, illustrated in the accompanying drawings. Wherever convenient, the same reference numbers will be used throughout the drawings to refer to the same or like parts. In the following description, reference is made to the accompanying drawings that form a part thereof, and in which is shown by way of illustration specific exemplary examples in which the invention may be practiced. These examples are described in sufficient detail to enable those skilled in the art to practice the invention and it is to be understood that other examples may be utilized and that changes may be made without departing from the scope of the invention. The following description is, therefore, merely exemplary.

Some embodiments solve the problem of identifying a metal alloy sample that falls within the limits of a given chemical composition specification yet is not from the indicated source of the metal alloy sample. Some embodiments thus solve the problem of identifying a metal alloy sample that is allegedly from a specified source, but that may have different physical properties as compared to metal alloy samples that are actually from the specified source.

According to some embodiments, a metal alloy source may have an associated unique metal alloy source fingerprint. The metal alloy source fingerprint may be determined using subtle properties in the chemical composition of metal alloy batches produced by the metal alloy source. While different metal alloy sources may produce metal alloys that all fall within specification limits for elemental composition, batches from the different metal alloy sources may still have small but significant differences in chemical composition. For example, trace elements that are allowable and present in metal alloy batches from one source may be absent from batches of the same metal alloy from another source. As another example, common elements may be present, but at different percentages within the allowable specification limits among batches from different metal alloy sources. According to some embodiments, metal alloy source fingerprints use such small differences in chemical composition-which may be within allowable chemical composition limits among various metal alloy sources-to characterize metal alloy samples produced by different metal alloy sources.

Some embodiments utilize metal alloy source fingerprints as proxies for non-chemical-composition properties and/or metal alloying processes. According to some embodiments, a metal alloy source fingerprint for a metal alloy sample may be determined by only the chemical composition of the sample, but may uniquely characterize not only a metal alloy source of the sample, but also non-chemical-composition properties of the sample and/or a metal alloying process used to create the sample. On the one hand, if a metal alloy sample that is allegedly from a particular metal alloy source matches the metal alloy source fingerprint of the particular metal alloy source, then the recipient of the metal alloy batch from which the metal alloy sample was obtained may be assured that the metal alloy batch was produced according to the certified process of the metal alloy source and thus complies with both chemical composition and non-chemical-composition requirements. The recipient may thus avoid having to subject the metal alloy batch to extensive, expensive, and time-consuming testing. On the other hand, if the metal alloy sample does not match the metal alloy source fingerprint for the metal alloy source that it allegedly originated from, then the recipient may reject the metal alloy batch from which the metal alloy sample was taken, or may subject the metal alloy batch to extensive, expensive, and time consuming testing, such as any, or a combination, of: quantitative chemical analysis by chemical titration, automated fatigue testing, ductility testing, tensile testing, X ray crystallography, electron microscopy, etc.

Some embodiments may use machine learning to group, associate, or cluster chemical composition quantifications for metal alloy samples that originate from a specified source. A cluster may represent a metal alloy source fingerprint that is associated with a specified supplier. Some embodiments may thus associate chemical composition quantifications for a (relatively narrow) range of different compositions that originate from a particular source. According to some embodiments, each source, such as a particular supplier or a particular facility for a supplier with multiple facilities, may be associated with a respective metal alloy source fingerprint that covers a respective range of chemical compositions from among batches produced by that source.

Some embodiments utilize supervised, semi-supervised, or unsupervised machine learning techniques to analyze metal alloy chemical compositions. Some embodiments utilize semi-supervised or unsupervised machine learning techniques to build clusters of chemical compositions associated with each source. Each cluster may be associated with, define, or characterize a metal alloy source fingerprint for a given source. When a new metal alloy sample needs to be analyzed, a new chemical composition quantification for the sample may be analyzed by the machine learning system to see if it fits in one of those clusters, and whether the cluster it fits in corresponds to the source that the sample supposedly originated from. If it does not fit in the cluster associated with its indicated source, the sample may be flagged, e.g., for further analysis or other action. For example, if a metal alloy sample does not match the metal alloy source fingerprint for its indicated source, it may be subjected to testing to determine if it fully complies with both chemical composition and non-chemical-composition requirements, whereas if it does match, such testing may be avoided.

Various embodiments may use any of a variety of techniques to derive a chemical composition quantification from a given metal alloy sample. By way of non-limiting example, any of the following may be used: X-ray fluorescence, inductively coupled plasma, optical emission spectrometry, mass spectrometry, atomic absorption spectrometry, electron probe micro-analysis, secondary ion mass spectrometry, inert gas fusion, etc. Some embodiments may use a combination of two or more of the aforementioned techniques.

Some embodiments provide a portable hand-held device that may obtain a chemical composition quantification using X-ray fluorescence and provide the chemical composition quantification for source analysis. According to some embodiments, the portable hand-held device may wirelessly transmit a chemical composition quantification to a system that conducts the source analysis. According to some embodiments, the portable hand-held device may be fully integrated; according to some embodiments, the portable hand-held device may not only obtain a chemical composition quantification for a metal alloy sample, but also analyze the chemical composition quantification to identify an associated metal alloy source and display an identification of the associated metal alloy source.

Some embodiments provide smart, adaptable, and continuously updated metal alloy analyses. According to some embodiments, as new chemical composition quantification data and its associated source analysis comes in, data from properly-identified material may be fed back into the machine learning model to allow the system to continuously learn and improve. Some embodiments may utilize chemical composition quantifications and analysis according to unsupervised or semi-supervised machine learning techniques to train supervised machine learning techniques.

Thus, some embodiments solve the prior art problem of detecting whether a metal alloy sample, which may fall within the limits of a given specification for the metal alloy, has different physical properties as compared to previous homogenous batches from the alleged source of the metal alloy sample and/or was produced using a different metal alloying process than was certified for the alleged source of the metal alloy sample. Some embodiments solve this problem using a machine learning system that is trained with a chemical composition quantification training corpus, where the chemical composition quantification training corpus includes training chemical composition quantifications characterizing training metal alloy samples, which may be within a chemical composition specification, that originate one or more metal alloy sources. Some embodiments solve this prior art problem through the use of metal alloy source fingerprints that characterize metal alloy sources and/or their certified metal alloying processes.

These and other features and advantages are disclosed presently in reference to the accompanying figures.

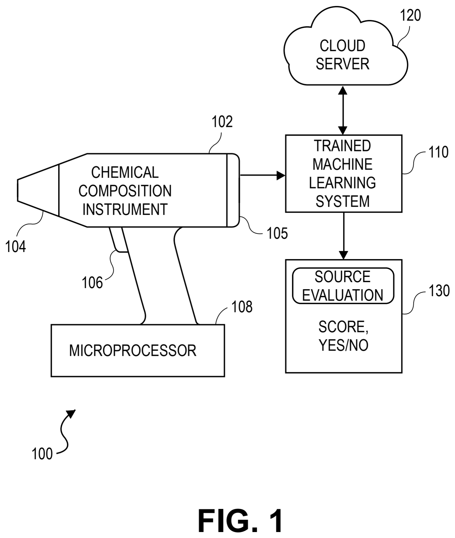

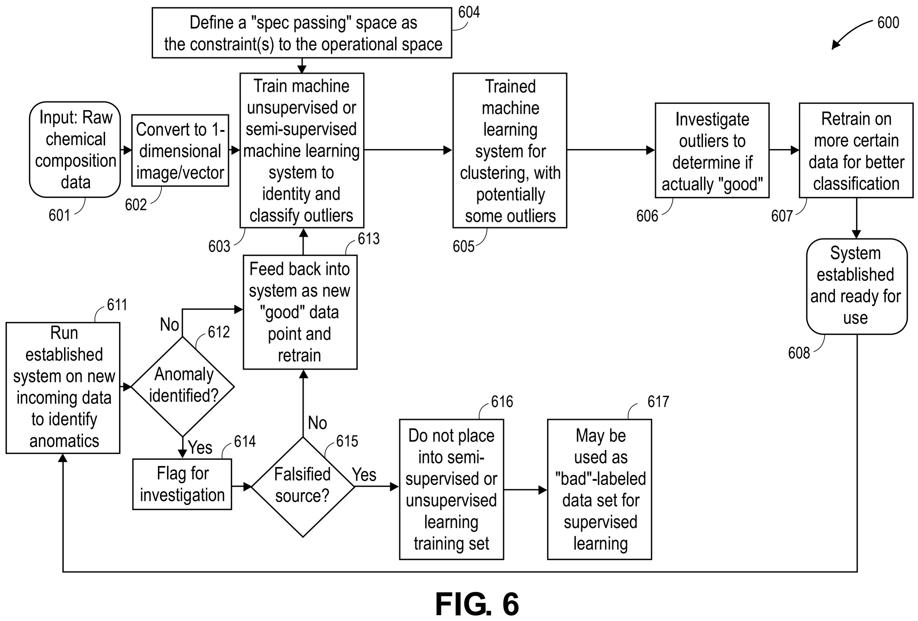

Fig. 1 is a schematic diagram of a system 100 for evaluating a metal alloy sample, according to various embodiments. The system 100 includes a chemical composition instrument 102 and a trained machine learning system 110. The system 100 may also include or utilize a cloud server 120, which may store data for, or otherwise facilitate, any of the methods disclosed herein. For example, the cloud server 120 may store data used to update the system 100, as shown and described herein in reference to Fig. 6. The system 100 provides a source evaluation 130 for a metal alloy sample to which the system 100 is applied.

The source evaluation 130 produced by the system 100 may be in any of various forms. According to some embodiments, the source evaluation 130 may be in the form of a score that indicates the likelihood that an analyzed metal alloy sample originated from a specified metal alloy source. According to some embodiments, the source evaluation 130 may include a plurality of scores, each representing a respective likelihood that the metal alloy sample originated from a respective metal alloy source from among a plurality of specified metal alloy sources. According to some embodiments, the source evaluation 130 may be in the form of a binary value (e.g., "YES," or "NO") indicative of whether the analyzed metal alloy sample originated from a specified metal alloy source. According to some embodiments, the source evaluation 130 may include a plurality of binary values, each indicative of whether the metal alloy sample originated from a respective metal alloy source of a plurality of specified metal alloy sources.

To perform its analysis, the chemical composition instrument 102 gathers raw chemical composition data from a material such as a metal alloy sample. By way of illustration, the chemical composition instrument 102 may be implemented using a portable hand-held X-ray fluorescence spectrometer that obtains raw chemical composition data in the form of spectroscopic data, according to some embodiments.

The chemical composition instrument 102 is not limited to X-ray fluorescence spectrometers. Other possible implementations of the chemical composition instrument 102 include, by way of non-limiting examples: an inductively coupled plasma instrument (which may obtain raw chemical composition data in the form of spectroscopic data), an optical emission spectrometer (which may obtain raw chemical composition data in the form of spectroscopic data), a mass spectrometer (which may obtain raw chemical composition data in the form of mass spectrum data), an atomic absorption spectrometer (which may obtain raw chemical composition data in the form of spectroscopic data), an electron probe microanalyzer (which may obtain raw chemical composition data in the form chemical composition data), a secondary ion mass spectrometer (which may obtain raw chemical composition data in the form of spectroscopic data), or an inert gas fusion instrument (which may obtain raw chemical composition data in the form of inert gas level data).

The chemical composition instrument 102 includes a microprocessor 108, which may perform all or part of any of the methods disclosed herein. The chemical composition instrument 102 may include a display 105, which may present the output or intermediate data of any of the methods disclosed herein. For example, according to some embodiments, the chemical composition instrument 102 may be fully integrated, including the trained machine learning system 110 as well as a display 105 on which the source evaluation 130 and an identification of the respective metal alloy source(s) considered in the source evaluation 130 may be presented. The chemical composition instrument 102 may be operated using a trigger 106, which may initiate gathering raw chemical composition data from a sample at which the chemical composition instrument 102 is aimed and/or analysis of such raw chemical composition data, as described in detail herein. The chemical composition instrument 102 may also include a nozzle 104, which collimates or focuses the X-ray beam onto a specific area of the sample from which the raw chemical composition data is obtained.

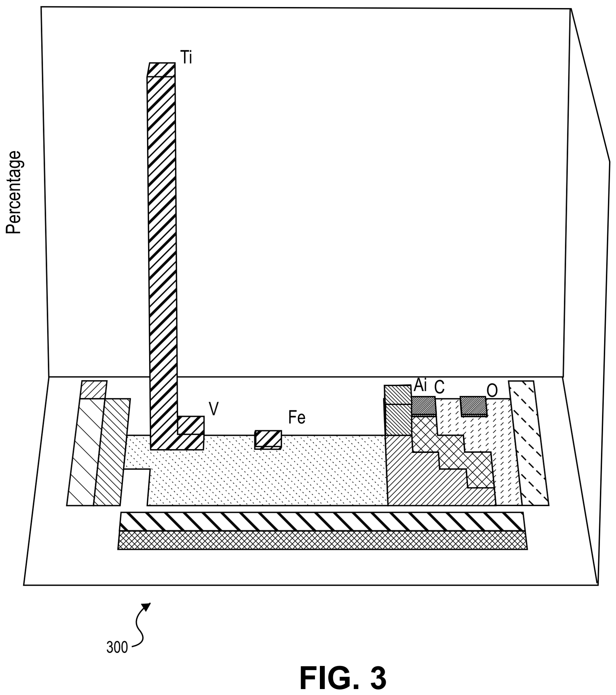

The trained machine learning system 110 accepts and processes a chemical composition quantification obtained from the raw chemical composition data gathered by the chemical composition instrument 102. A non-limiting example of a chemical composition quantification is an element quantification in the form of a vector that includes values indicative of amounts of each of a plurality of chemical elements. Additional details and specific examples of chemical composition quantifications are shown and described herein in reference to Figs. 3-5. The derivation of the chemical composition quantification from the raw chemical composition data may be performed by the chemical composition instrument, by the trained machine learning system 110, or by a different system. Details of such derivation are shown and described herein in reference to Figs. 3-5.

To perform a source analysis of a metal composition quantification, the trained machine learning system 110 may implement an unsupervised machine learning technique, a semi-supervised machine learning technique, a supervised machine learning technique, or a combination of any of the aforementioned machine learning techniques. Examples of such techniques are described in detail presently.

Embodiments that use unsupervised or semi-supervised machine learning may be advantageous in some circumstances. For example, sufficient labeled training data for supervised machine learning may be unavailable, because training data corresponding to the huge range of possible scenarios for metal alloys samples with incorrect source indications may be difficult to obtain.

Accordingly, some embodiments may use unsupervised or semi-supervised machine learning clustering techniques, by way of non-limiting example. Further by way of non-limiting example, such clustering techniques may include: k means clustering, k-medoids clustering, agglomerative clustering, or Gaussian Mixture Models (GMM). k-means clustering may be particularly effective because a number of clusters corresponding to the number of sources may be specified. These techniques may be trained using a training corpus of training data that may include chemical composition quantifications for metal alloy samples that are known to be fully compliant with the chemical composition specification for the metal alloy. Each datum may be unlabeled or may be labeled with the corresponding source. The clustering techniques may generate a projection from a space with the dimensionality of the chemical quantifications (e.g., the number of elements represented, which may be all or part of the periodic table of elements) to a reduced dimensional space (e.g., two-dimensional or three-dimensional). The training data may be projected into the reduced dimensional space, and a plurality of clusters in the reduced dimensional space may be formed, where each cluster corresponds a metal alloy source. Such clusters may be representative of metal alloy source fingerprints. Once trained, a new chemical composition quantification for a new metal alloy sample may be analyzed, e.g., by projecting it into the reduced dimensional space using the trained machine learning system and determining a matching cluster, or lack thereof. If the new chemical composition quantification does not match any of the clusters that represent certified metal alloy sources, then the metal alloy sample may be marked as "suspect" or "source incorrectly indicated."

Here, a match between a new chemical composition quantification and a cluster could correspond to any of a variety of conditions, such as, by way of non-limiting example: the projection of the new chemical composition quantification into the reduced dimensional space falls within the cluster, the projection is within a specified number of standard deviations from the cluster center of mass of the cluster, the new chemical composition quantification is classified as being within the cluster by the trained machine learning system, etc. Whether or not a match occurs for a cluster that corresponds to a metal alloy source fingerprint may be used to provide a source evaluation of the metal alloy sample with respect to the metal alloy source, e.g., in the form of a binary value reflective of whether or not the match occurs. According to some embodiments, a distance between the projection of the chemical composition quantification and the center of mass of the cluster may be converted into a source evaluation in the form of a score, e.g., by using the distance itself as the score or normalizing the distance prior to presenting it as a score. Such a score may characterize a likelihood of whether the metal alloy sample originated from the metal alloy source and was produced using the certified alloying process of the meal alloy source. The score may be parsed into intervals that correspond to, for example, "authentic source identification," "questionable source identification," and "inauthentic source authentication." According to some embodiments, both a binary value and a score may be presented as a source evaluation with respect to a specified source, where the score may be interpreted as a confidence of the accuracy for the binary value. Note that although the aforementioned described providing a source evaluation (e.g., a binary value and/or a score) for one metal alloy source, multiple metal alloy sources may be considered and respective source evaluations for each considered metal alloy source may be provided.

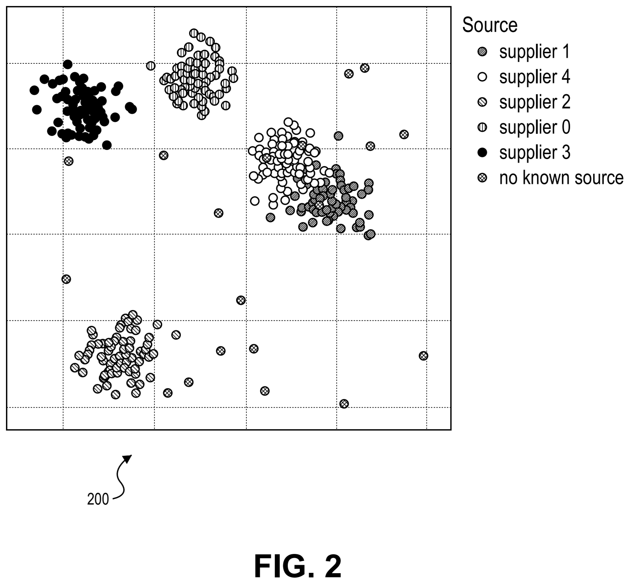

Fig. 2 depicts clusters of chemical composition quantifications projected into a reduced dimensional space 200, according to various embodiments. In the example of Fig. 2, the reduced dimensional space 200 is two-dimensional, represented by a plane. Each dot in Fig. 2 represents the projection of a chemical composition quantification into the reduced dimensional space 200. Each cluster represents a different metal alloy source, e.g., a different metal alloy supplier or facility for a supplier with multiple facilities. Note that although the clusters may partially overlap, in general, each cluster may be disjointed from other clusters. Spread throughout the reduced dimensional space are dots representing projections of chemical composition quantifications for metal alloy samples that are not from any of the metal alloy sources identified in Fig. 2.

Each cluster may represent, characterize, or define a respective metal alloy source fingerprint. If a given dot matches a particular cluster, then the dot may represent a metal alloy sample that was produced by the respective metal alloy source of that particular cluster using the certified metal alloying process of the metal alloy source. Various embodiments may identify whether a particular dot matches a particular cluster in any of a variety of ways, including classification by a trained unsupervised machine learning system as presented herein.

Other unsupervised and semi-supervised machine learning techniques include anomaly detection techniques. By way of illustration rather than limitation, example anomaly detection techniques include single-class support vector machines and isolation forests. A single-class support vector machine may generate a hyperplane or other boundary in a high-dimensional space, where one side of the boundary represents chemical composition quantifications from metal alloy samples that are from a specified source, and where the other side of the boundary represents chemical composition quantifications for metal alloy samples that are not from the specified source, i.e., that are anomalous. A new chemical composition quantification may be represented in the space, and a determination of on which side of the boundary the representation lies may be used as an indication of whether the corresponding metal alloy sample originated from the specified source. Multiple single-class support vector machines may be included in some embodiments in order to determine whether a given chemical composition sample is from, or anomalous with respect to, any of multiple specified metal alloy sources. Isolation forests are also suitable anomaly detection techniques that may be trained in an unsupervised or semi-supervised manner.

In general regarding semi-supervised and unsupervised machine learning techniques, training with a training data corpus that includes only known-good inputs with labels that dictate which source provided the metal alloy samples may be appropriate. Semi-supervised and unsupervised machine learning techniques may be sensitive to outliers (samples with incorrect or falsified source labeling) that are included during training, which could reduce the effectiveness of such techniques. According to some embodiments, possible outliers identified during training may be flagged for investigation to see if they were incorrectly labeled, and the metal alloy qualifications for those outliers (if any) may be removed from the training corpus for the machine learning system for re-training. Further details of this latter process are shown and described herein in reference to Fig. 6.

Supervised machine learning techniques that may be used according to various embodiments include neural networks, decision trees, random forests, XGBoost, and other techniques. Supervised machine learning techniques may utilize a training corpus of training chemical composition quantifications that are labeled. Each label may identify a source, e.g., supplier or facility. Each label may also indicate whether the source of the associated metal alloy sample was correctly or incorrectly indicated. According to some embodiments, data with inaccurate source indications may be removed from the training corpus before using it to train the machine learning system, particularly for unsupervised and semi-supervised machine learning systems.

Over time, embodiments may detect that the source indications for many metal alloy samples are incorrect. These detections may be made using embodiments that include unsupervised, semi-supervised, and/or supervised machine learning systems. The chemical composition quantifications for these samples may be stored, e.g., in the cloud server 120, labeled appropriately, and included in a training corpus that is used to train or retrain unsupervised, semi-supervised, or supervised machine learning systems. According to some embodiments, an unsupervised or semi-supervised embodiment may be used to determine whether source indications for metal alloy samples are correct or incorrect, and over time, the chemical composition quantifications for those with incorrect source indications as determined using semi-supervised or unsupervised machine learning may be added to the training corpus used to train a supervised machine learning system.

Regarding re-training according to some embodiments, some embodiments stay current using the cloud server. For example, the chemical composition instrument or other system portion for implementations throughout the world may upload new data to the cloud system. The new data may include chemical composition quantifications, with or without labels, and may be anonymized. A centralized server may retrain the machine learning systems periodically as new data is stored in the cloud server. Further, updated trained machine learning systems may be pushed to devices, such as integrated chemical composition instruments, e.g., during Wi-Fi sync. Thus, some embodiments incorporate adaptive learning in which the overall system evolves to catch new types of misidentified metal alloys, even metal alloys specifically designed to bypass older system versions.

Fig. 3 depicts a non-limiting example chemical composition quantification 300, shown pictorially for purposes of illustration over a periodic table, according to various embodiments. The suggestive illustration of Fig. 3 shows various elevations over the locations of the elements on the periodic table of elements, where the heights of the elevations correspond to the amount of the respective elements in the metal alloy sample represented by the chemical composition quantification 300.

In an embodiment, a chemical composition quantification may be represented by a data structure, e.g., stored in a non-transitory computer-readable medium. The data structure may be a one-dimensional vector, for example. In order to standardize the vector for input into the various machine learning systems, it may be formatted in such a way so that positions in the vector correspond to elements, based on atomic number for example (e.g. hydrogen in the first position, helium in the second position, lithium in the third position, etc.). An example of such positions is shown below.

[H, He, Li, Be, B, C, N, O, F, Ne, Na, Mg, Al, Si, P, S, Cl, Ar, Ar, Ca, Sc, Ti, V, Cr, Mn, Fe, Co, Ni, Cu, ..., Og]
An advantage of including every possible element (no matter how unlikely) instead of just those detected allows for every vector to be the same size and shape, for consistent input into the machine learning system. The value at a position in the vector corresponds to the amount of that element present. The amount can be recorded in any of several types, such as percentage by weight, mole fraction, mole ratio, mass fraction, mass ratio, parts per million (or billion), etc., but should be consistent within a training corpus. An example element quantification vector that represents the chemical composition quantification 300 illustrated in Fig. 3 is presented below.

[0, 0, 0, 0 , 0, 0.02, 0, 0.15, 0, 0, 0, 0, 6.0, 0, 0, 0, 0, 0, 0, 0, 0, 89.0, 4.0, 0, 0, 0.21, 0, 0, 0, ..., 0]
The presented example vector is for a theoretical titanium alloy that could be compatible with the AMS4954 specification, with roughly 89% titanium, 6% aluminum, 4% vanadium, 0.21% iron, 0.15% oxygen, 0.02% carbon, and 0% for all other elements by weight. Although a titanium alloy is illustrated in the examples, such evaluation method described herein may be used for many other alloys such as aluminum, magnesium, ferrous (including maraging steel and alloy steel), copper, nickel, and the like, and including super alloys.

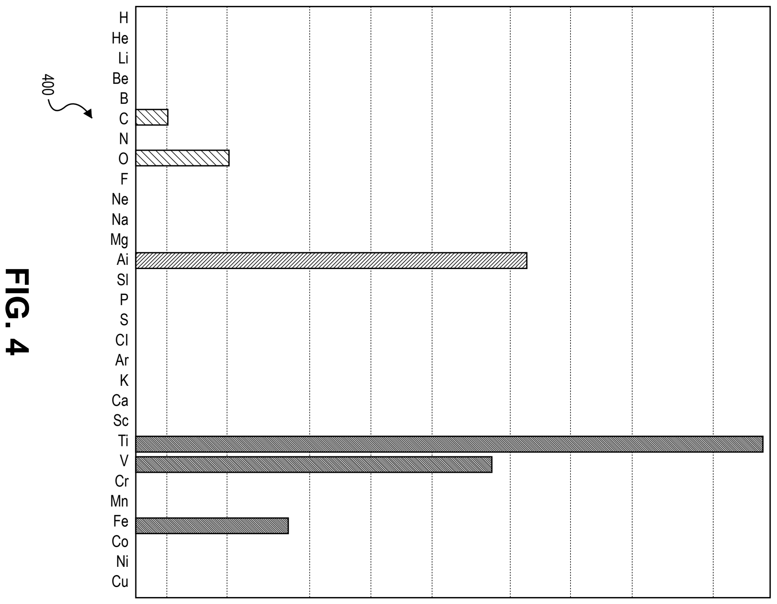

Fig. 4 depicts a portion of a chemical composition quantification 400, shown pictorially for purposes of illustration as a bar chart, according to various embodiments. The chemical composition quantification 400 is for the same chemical composition quantification 300 shown and described in reference to Fig. 3, but is shown in the form of a bar chart strictly for illustrative purposes.

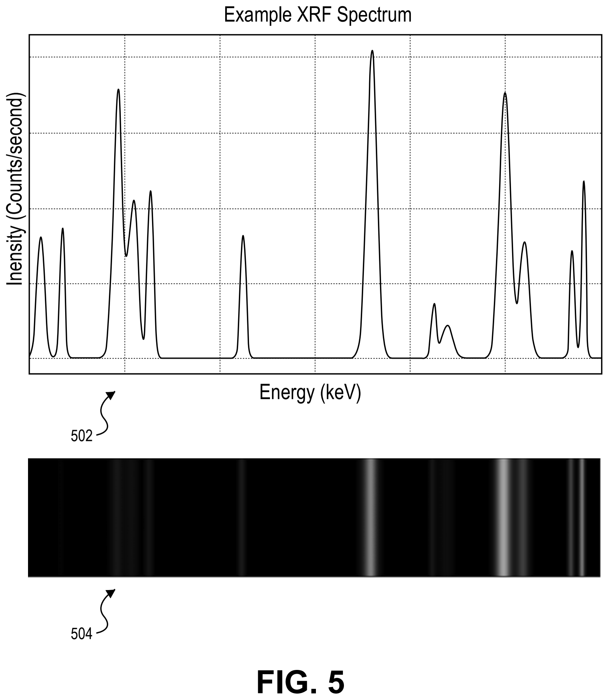

The chemical composition quantifications according to various embodiments may be derived from raw chemical composition data, e.g., as obtained by the chemical composition instrument 102 as shown and described in reference to Fig. 1. The raw chemical composition data may be in any of a variety of domains, not limited to spectroscopic data. Further, the raw chemical composition data may have any of a variety of formats. Fig. 5 illustrate an example domain and format, by way of non-limiting example.

Fig. 5 depicts a portion of raw chemical composition data in domain of X-ray fluorescence and in the forms of a spectrogram line graph 502 and a spectrogram color intensity graph 504, according to various embodiments. The X-ray fluorescence spectrogram line graph 502 and the spectrogram color intensity graph 504 represent the same example metal alloy.

Some embodiments may obtain chemical composition quantifications by processing raw chemical composition data to account for differences in resolution, range, format, or other properties. Thus, according to some embodiments, a chemical composition quantification could be in the form of a processed image of a spectrum such as the X-ray fluorescence spectrogram line graph 502 and a spectrogram color intensity graph 504. For example, such a chemical composition quantification may be in the form of an image, such as a processed form of the X-ray fluorescence spectrogram line graph 502 of spectrogram color intensity graph 504. As another example, such a chemical composition quantification may be in the form of a vector similar to that described herein in reference to Fig. 3, except that positions in the vector may be dictated by the x-axis of the spectrum (e.g., energy as in the X-ray fluorescence spectrogram line graph 502, angle in an X-ray diffraction spectrum, etc.) instead of atomic number. Values for each position may be determined according to y-axis values at the corresponding locations along the X-ray fluorescence spectrogram line graph 502.

Processing the raw chemical composition data in the X-ray spectroscopy domain, by way of non-limiting example, in order to obtain a chemical composition quantification, may include ensuring that no matter which spectrum is used, the raw chemical composition data is converted or translated so that the positions in the vector of the chemical composition quantification correspond to the same values across the data set, e.g., training data corpus. That is, the processing may include generating a quantized spectrogram in a standardized format. The quantized spectrogram may be derived from spectroscopic data and standardized to a common format. The quantized spectrogram may be a vector derived from spectroscopic data and standardized to a common format, wherein positions in the vector correspond to energy levels of a spectrum. For example, if a first dataset representative of spectroscopic data has an x-axis in energy that goes from 1 keV to 10 keV, with a resolution of 0.1 keV, and a second dataset's x-axis (again in energy) goes from 2 keV to 12 keV with a resolution of 0.2 keV, and the space of all datasets ranges from 0 keV to 40 keV, by way of non-limiting example for an embodiment, then following may be performed as at least part of processing the raw chemical composition data into chemical composition quantifications:
- The first dataset may have zeroes added from 10 to 40 keV.
- The second dataset may have zeroes added from 1 to 2 keV and from 12 to 40keV.
- A standard resolution may be established:
   ∘ If the standard resolution is 0.1 keV:
      ▪ No change is needed for first dataset.
      ▪ The second dataset may have interpolation performed to fill in gaps between 0.2 keV steps.
   ∘ If the standard resolution is 0.2 keV:
      ▪ The first dataset may undergo down-sampling to 0.2 keV resolution (e.g., averaging nearest neighbors, rolling average, linear interpolation, quadratic interpolation, etc.).
      ▪ No change is needed for the second dataset.
   ∘ If the standard resolution is 0.15 keV:
      ▪ The first dataset may undergo down-sampling to 0.15 keV resolution (e.g., averaging nearest neighbors, rolling average, linear interpolation, quadratic interpolation, etc.)
      ▪ The second dataset may have interpolation performed to reach 0.15 keV steps (e.g. interpolate three points in between each 0.2 keV step, and select every value that lies on a 0.15 keV multiple whether it is a real value or an interpolated value).

Note that embodiments are not limited to raw chemical composition data in the domain of spectroscopic data. For example, according to some embodiments, the raw spectroscopic data may be in the X-ray diffraction domain, e.g., in the form of an X-ray diffraction pattern. Such an X-ray diffraction pattern may represent raw chemical composition data that may be converted to a chemical composition quantification in the form of an image, a spectrum, a series of spectra taken at various angles of the X-ray diffraction pattern, or as such spectra converted to vectors in a standardized format such as described herein.

As described above in reference to Fig. 5, chemical composition quantifications in the form of quantized spectrograms or quantized diffraction domains may be obtained from raw chemical composition data such as spectrograms and X ray diffraction data, respectively. The quantized spectrogram may be derived from spectroscopic data and standardized to a common format. The quantized spectrogram may be a vector derived from spectroscopic data and standardized to a common format, wherein positions in the vector correspond to energy levels of a spectrum. Alternately, chemical composition quantifications in the form of element quantification vectors may be obtained from spectrograms and X ray diffraction data using spectral deconvolution techniques, for example.

Note that chemical composition quantifications according to various embodiments may not necessarily expressly depict elemental composition, but instead include representations of other aspects that can be used to build the metal alloy source fingerprint of a supplier or facility. Yet another example of a chemical composition quantification may include a ratio of different types of crystal structure in the microstructure (e.g. the ratio of alpha to beta grains in a titanium alloy).

Fig. 6 is a flow chart for a method 600 of training and using a machine learning system for evaluating a metal alloy sample, according to various embodiments. The method 600 may use a machine learning system such as is shown and described herein in reference to Fig. 1. For purposes of illustration rather than limitation, the method 600 may be conceptually divided into a training phase (601, 602, 603, 604, 605, 606, 607, 608) and a deployment phase (611, 612, 613, 614, 615, 616, 617).

At 601, raw chemical composition data for a plurality of training metal alloy samples is obtained. Any number of metal alloy samples may be represented, but large numbers of samples from multiple sources are expected to produce a more accurate system. At 602, the raw chemical composition data is converted into chemical composition quantifications. By way of non-limiting example, the chemical composition quantifications may be in the form of one-dimensional vectors. In general, any of the processes described in reference to any of Figs. 3-6 may be used for the conversion. The actions of 602 produce a training corpus of chemical composition quantifications. At 603, an unsupervised or semi-supervised machine learning system may be trained using the chemical composition training corpus. The actions of 603 may be as described herein in reference to Figs. 1 and 2, for example. Thus, the unsupervised or semi-supervised machine learning system may include machine learning clustering, a single-class support vector machine, or an isolation forest, by way of non-limiting examples. The actions of 603 generate a trained machine learning system 605. The trained machine learning system 605 may represent one or more outliers that were present in the chemical composition training corpus. Because semi-supervised and unsupervised machine learning techniques can be sensitive to outliers in their training corpus, which could reduce their accuracy in the deployment phase, at 606 any potential outliers are investigated. If any such potential outliers are good, e.g., in the sense that their source is correctly indicated, they may be preserved in the training corpus. Otherwise, they may be removed from the training corpus, and at 607, the machine learning system may be re-trained with the reduced training corpus. These actions produce a trained machine learning system, ready for deployment.

During deployment, at 611, the trained machine learning system is provided with a chemical composition quantification for a new metal alloy sample to obtain a quantitative source estimation representative of its source according to the trained machine learning system. The quantitative source estimation may include one or more scores or one or more binary values, according to various embodiments, as described herein reference to Fig. 1, for example. The quantitative source estimation may be indicative of whether the new metal alloy sample is anomalous with respect to one or more metal alloy source fingerprints, as gauged by the trained machine learning system. If not, then at 613, chemical composition quantification for the new metal alloy sample may be added to the training corpus used to train the machine learning system. The machine learning system may be periodically retrained as the training corpus is expanded. If the new metal alloy sample is anomalous with respect to one or more metal alloy source fingerprints (e.g., for a specified metal alloy source or for all of the metal alloy sources represented by the trained machine learning system), then at 614, the metal alloy sample may be flagged. The flag may represent that the metal alloy sample does not match any of the represented metal alloy source fingerprints and thus may not have been produced according to a certified process. Because the metal alloy source fingerprints may act as proxies for non-chemical-composition properties and/or metal alloying processes, the flag may indicate that the new metal alloy sample should be subjected to advanced chemical component testing and/or non-chemical component testing at 615, such as any, or a combination, of: quantitative chemical analysis by chemical titration, automated fatigue testing, ductility testing, tensile testing, X ray crystallography, electron microscopy, etc. If the testing indicates that the new metal alloy sample is compliant with all required specifications, then its chemical composition quantification may be added to the training corpus at 613. Otherwise, if the testing confirms that the alleged source of the new metal alloy sample has been falsified, then at 616, the chemical composition quantification for the new metal alloy sample may be excluded from the training corpus, at least with respect to the usage of the training corpus to train a semi-supervised or unsupervised machine learning system. Nevertheless, at 617, the chemical composition quantification for the new metal alloy sample may be preserved, e.g., by storage in the cloud server 120, labeled as being indicative of an anomaly, and included in a training corpus used to train a supervised machine learning system, such as a supervised machine learning system as described herein in reference to Fig. 1.

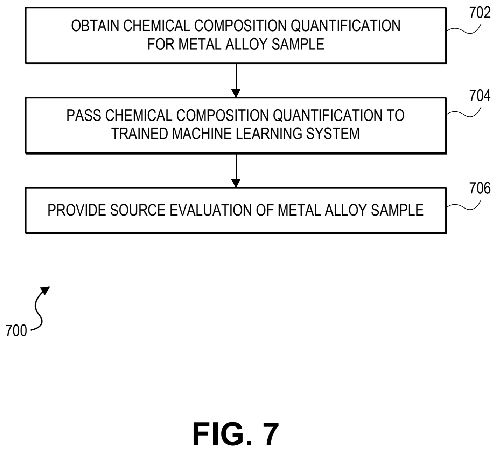

Fig. 7 is a flow diagram for a method 700 of evaluating a metal alloy sample, according to various embodiments. The method 700 may be practiced using a system such as is shown and described herein in reference to Fig. 1. The method 700 may include any of the actions of the method shown and described herein in reference to Fig. 6.

At 702, the method 700 includes obtaining a chemical composition quantification for a metal alloy sample. The metal alloy sample may have been obtained from an entity who alleged that the metal alloy sample originated from a specified certified metal alloy source. The metal alloy sample may have been obtained from a batch of the metal alloy provided by the entity to a manufacturer in any of a variety of industries.

According to various embodiments, the chemical composition quantification may be any of the possibilities as shown and described in reference to any of the preceding figures. For example, the chemical composition quantification may be obtained from raw chemical composition data obtained using any of a variety of techniques as disclosed herein in reference to any of the preceding figures. By way of non-limiting example, any of X-ray fluorescence, inductively coupled plasma, optical emission spectrometry, mass spectrometry, atomic absorption spectrometry, electron probe microanalyzer, secondary ion mass spectrometry, or inert gas fusion may be used to obtain the raw chemical composition data. According to some embodiments, the chemical composition quantification may be obtained from spectroscopic data provided by a portable hand-held X-ray fluorescence spectrometer applied to the metal alloy sample.

At 704, the method 700 includes passing the chemical composition quantification to a trained machine learning system, such as an unsupervised machine learning system, a semi-supervised machine learning system, or a supervised machine learning system. The trained machine learning system may be integrated into the chemical composition instrument that was used to obtain the raw chemical composition data, or may be communicatively coupled to the chemical composition instrument, for example. By way of non-limiting example, the trained machine learning system may include an anomaly detection machine learning system. The trained unsupervised machine learning system may have undergone machine learning training with a chemical composition quantification training corpus, where the chemical composition quantification training corpus includes a plurality of training chemical composition quantifications for training metal alloy samples that originate from one or more metal alloy sources. According to some embodiments, a respective metal alloying process may have been certified for each of the metal alloy sources, each having an associated respective metal alloy source fingerprint representing a respective metal alloying process. In response to passing the chemical composition quantification, the trained machine learning system may provide a quantitative source estimation. The quantitative source estimation may match, correspond to, or represent a metal alloy source fingerprint that characterizes a particular metal alloying process of the particular metal alloy source. The chemical composition quantification may be passed automatically from a chemical composition instrument or from a different system.

At 706, the method 700 includes providing a source evaluation of the metal alloy sample. The source evaluation of the metal alloy sample may be based on the quantitative source estimation. By way of non-limiting examples, the source evaluation of the metal alloy sample may be in the form of a binary value that corresponds to whether or not the metal alloy sample originated from the particular metal alloy source and was produced by a metal alloying process certified for the particular metal alloy source, or may be in the form of a score that characterizes a likelihood of whether the metal alloy sample originated from the particular metal alloy source and was produced by a metal alloying process certified for the particular metal alloy source. The source evaluation of the metal alloy sample may include (e.g., identify) a particular metal alloy source relative to which the quantitative source estimation was determined. The particular metal alloy source may be among the metal alloy source(s) from among which metal alloy samples were used to obtain training chemical composition quantifications. According to some embodiments, e.g., in which the trained machine learning system is trained using a clustering algorithm and the quantitative source estimation includes an identification of a closest cluster, the source evaluation of the metal alloy sample may include a score representative of a likelihood that the metal alloy sample belongs to the closest cluster. According to some embodiments, the source evaluation of the metal sample may include a plurality of scores representative of likelihoods that the metal alloy sample belongs to each of a plurality of previously identified clusters. The source evaluation may be provided by displaying it, e.g., on a display integrated into a chemical composition instrument, such as is shown and described herein in reference to Fig. 1.

According to some embodiments, the method 700 may further include retraining the trained machine learning system with a chemical composition quantification retraining corpus, where the chemical composition quantification retraining corpus includes the chemical composition quantification for the newly analyzed metal alloy sample.

Certain examples can be performed using a computer program or set of programs. The computer programs can exist in a variety of forms both active and inactive. For example, the computer programs can exist as software program(s) comprised of program instructions in source code, object code, executable code or other formats; firmware program(s), or hardware description language (HDL) files. Any of the above can be embodied on a transitory or non-transitory computer readable medium, which include storage devices and signals, in compressed or uncompressed form. Exemplary computer readable storage devices include conventional computer system RAM (random access memory), ROM (read-only memory), EPROM (erasable, programmable ROM), EEPROM (electrically erasable, programmable ROM), flash memory, and magnetic or optical disks or tapes.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented using computer readable program instructions that are executed by an electronic processor.

These computer readable program instructions may be provided to a processor of a general-purpose computer, special purpose computer, or other programmable data processing apparatus to produce a machine, such that the instructions, which execute via the electronic processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

In embodiments, the computer readable program instructions may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the C programming language or similar programming languages. The computer readable program instructions may execute entirely on a user's computer, partly on the user's computer, as a stand-alone software package, partly on the user's computer and partly on a remote computer or entirely on the remote computer or server.

As used herein, the terms "A or B" and "A and/or B" are intended to encompass A, B, or {A and B}. Further, the terms "A, B, or C" and "A, B, and/or C" are intended to encompass single items, pairs of items, or all items, that is, all of: A, B, C, {A and B}, {A and C}, {B and C}, and {A and B and C}. The term "or" as used herein means "and/or."

As used herein, language such as "at least one of X, Y, and Z," "at least one of X, Y, or Z," "at least one or more of X, Y, and Z," "at least one or more of X, Y, or Z," "at least one or more of X, Y, and/or Z," or "at least one of X, Y, and/or Z," is intended to be inclusive of both a single item (e.g., just X, or just Y, or just Z) and multiple items (e.g., {X and Y}, {X and Z}, {Y and Z}, or {X, Y, and Z}). The phrase "at least one of" and similar phrases are not intended to convey a requirement that each possible item must be present, although each possible item may be present.

The techniques presented and claimed herein are referenced and applied to material objects and concrete examples of a practical nature that demonstrably improve the present technical field and, as such, are not abstract, intangible or purely theoretical.

While the invention has been described with reference to the exemplary examples thereof, those skilled in the art will be able to make various modifications to the described examples without departing from the scope of the claims. The terms and descriptions used herein are set forth by way of illustration only and are not meant as limitations. In particular, although the method has been described by examples, the steps of the method can be performed in a different order than illustrated or simultaneously. Those skilled in the art will recognize that these and other variations are possible within the scope as defined in the following claims.

The disclosure also includes the following clauses:
1. A method (600, 700) of evaluating a metal alloy sample, the method comprising:
   Obtaining (702) a chemical composition quantification (300, 400) for the metal alloy sample, wherein the chemical composition quantification is obtained from spectroscopic data provided by a portable hand-held X-ray fluorescence spectrometer applied to the metal alloy sample;
   passing (704), automatically, the chemical composition quantification to a trained machine learning system (110, 605), from which a quantitative source estimation is obtained, wherein the trained machine learning system is trained with a chemical composition quantification training corpus, wherein the chemical composition quantification training corpus comprises a plurality of training chemical composition quantifications characterizing training metal alloy samples that originate from a plurality of metal alloy sources; and
   providing (706) a source evaluation (130) of the metal alloy sample, wherein the source evaluation of the metal alloy sample is based on the quantitative source estimation, and wherein the source evaluation of the metal alloy sample is relative to a particular metal alloy source.
2. The method of clause 1, wherein the portable hand-held X-ray fluorescence spectrometer comprises the trained machine learning system.
3. The method of clause 2, wherein the providing comprises providing on a display (105) integrated into the portable hand-held X-ray fluorescence spectrometer.
4. The method of any one of clauses 1-3, wherein the trained machine learning system is communicatively coupled to the portable hand-held X-ray fluorescence spectrometer, and wherein the passing comprises automatically wirelessly transmitting.
5. The method of any one of clauses 1-4, wherein the source evaluation of the metal alloy sample comprises one of a binary value, wherein the binary value corresponds to whether or not the metal alloy sample originated from the particular metal alloy source and was produced by a metal alloying process certified for the particular metal alloy source.
6. The method of any one of clauses 1-5, wherein the source evaluation of the metal alloy sample comprises a score, wherein the score characterizes a likelihood of whether the metal alloy sample originated from the particular metal alloy source and was produced by a metal alloying process certified for the particular metal alloy source.
7. The method of any one of clauses 1-6, wherein the quantitative source estimation matches a metal alloy source fingerprint that represents a particular metal alloying process of the particular metal alloy source.
8. The method of any one of clauses 1-7, wherein the trained machine learning system is trained using a clustering algorithm, wherein the quantitative source estimation comprises an identification of a closest cluster, and wherein the source evaluation of the metal alloy sample comprises a score representative of a likelihood that the metal alloy sample belongs to the closest cluster.
9. The method of any one of clauses 1-8, further comprising retraining (613) the trained machine learning system with a chemical composition quantification retraining corpus, wherein the chemical composition quantification retraining corpus comprises the chemical composition quantification.
10. The method of any one of clauses 1-9, wherein the source evaluation of the metal sample comprises a plurality of scores representative of likelihoods that the metal alloy sample belongs to each of a plurality of previously identified clusters.
11. A system (100) for evaluating a metal alloy sample, the system comprising:
   a portable hand-held X-ray fluorescence spectrometer configured to obtain spectroscopic data when applied to the metal alloy sample;
   a non-transitory computer readable medium comprising instructions; and
   at least one electronic processor (108) that executes the instructions to perform operations comprising:
      obtaining (702) a chemical composition quantification (300, 400) for the metal alloy sample, wherein the chemical composition quantification is obtained from the spectroscopic data;
      passing (704), automatically, the chemical composition quantification to a trained machine learning system (110, 605), from which a quantitative source estimation is obtained, wherein the trained machine learning system is trained with a chemical composition quantification training corpus, wherein the chemical composition quantification training corpus comprises a plurality of training chemical composition quantifications characterizing training metal alloy samples that originate from a plurality of metal alloy sources; and
      providing (706) a source evaluation (130) of the metal alloy sample, wherein the source evaluation of the metal alloy sample is based on the quantitative source estimation, and wherein the source evaluation of the metal alloy sample comprises a particular metal alloy source.
12. The system of clause 11, wherein the portable hand-held X-ray fluorescence spectrometer comprises the trained machine learning system.
13. The system of clause 12, wherein the portable hand-held X-ray fluorescence spectrometer comprises an integrated display (105), and wherein the providing comprises providing on the display.
14. The system of any one of clauses 11-13, wherein the trained machine learning system is communicatively coupled to the portable hand-held X-ray fluorescence spectrometer, and wherein the passing comprises automatically wirelessly transmitting.
15. The system of any one of clauses 11-14, wherein the source evaluation of the metal alloy sample comprises one of a binary value, wherein the binary value corresponds to whether or not the metal alloy sample originated from the particular metal alloy source and was produced by a metal alloying process certified for the particular metal alloy source.
16. The system of any one of clauses 11-15, wherein the source evaluation of the metal alloy sample comprises a score, wherein the score characterizes a likelihood of whether the metal alloy sample originated from the particular metal alloy source and was produced by a metal alloying process certified for the particular metal alloy source.
17. The system of any one of clauses 11-16, wherein the quantitative source estimation matches a metal alloy source fingerprint that represents a particular metal alloying process of the particular metal alloy source.
18. The system of any one of clauses 11-17, wherein the trained machine learning system is trained using a clustering algorithm, wherein the quantitative source estimation comprises an identification of a closest cluster, and wherein the source evaluation of the metal alloy sample comprises a score representative of a likelihood that the metal alloy sample belongs to the closest cluster.
19. The system of any one of clauses 11-18, wherein the operations further comprise retraining (613) the trained machine learning system with a chemical composition quantification retraining corpus, wherein the chemical composition quantification retraining corpus comprises the chemical composition quantification.
20. The system of any one of clauses 11-19, wherein the source evaluation of the metal sample comprises a plurality of scores representative of likelihoods that the metal alloy sample belongs to each of a plurality of previously identified clusters.

## Claims

1. A method (600, 700) of evaluating a metal alloy sample, the method comprising:
obtaining (702) a chemical composition quantification (300, 400) for the metal alloy sample, wherein the chemical composition quantification is obtained from spectroscopic data provided by a portable hand-held X-ray fluorescence spectrometer applied to the metal alloy sample;
passing (704), automatically, the chemical composition quantification to a trained machine learning system (110, 605), from which a quantitative source estimation is obtained, wherein the trained machine learning system is trained with a chemical composition quantification training corpus, wherein the chemical composition quantification training corpus comprises a plurality of training chemical composition quantifications characterizing training metal alloy samples that originate from a plurality of metal alloy sources, thereby characterizing a metal alloy source fingerprint for each of the one or more metal alloy sources, the metal alloy source fingerprint representing an inherent chemical fingerprint; and
providing (706) a source evaluation (130) of the metal alloy sample, wherein the source evaluation of the metal alloy sample is based on the quantitative source estimation, and wherein the source evaluation of the metal alloy sample is relative to a particular metal alloy source.

2. The method of claim 1, wherein the portable hand-held X-ray fluorescence spectrometer comprises the trained machine learning system.

3. The method of claim 2, wherein the providing comprises providing on a display (105) integrated into the portable hand-held X-ray fluorescence spectrometer.

4. The method of any one of claims 1-3, wherein the trained machine learning system is communicatively coupled to the portable hand-held X-ray fluorescence spectrometer, and wherein the passing comprises automatically wirelessly transmitting.

5. The method of any one of claims 1-4, wherein the source evaluation of the metal alloy sample comprises one of a binary value, wherein the binary value corresponds to whether or not the metal alloy sample originated from the particular metal alloy source and was produced by a metal alloying process certified for the particular metal alloy source.

6. The method of any one of claims 1-5, wherein the source evaluation of the metal alloy sample comprises a score, wherein the score characterizes a likelihood of whether the metal alloy sample originated from the particular metal alloy source and was produced by a metal alloying process certified for the particular metal alloy source.

7. The method of any one of claims 1-6, wherein the quantitative source estimation matches the metal alloy source fingerprint that represents a particular metal alloying process of the particular metal alloy source.

8. The method of any one of claims 1-7, wherein the trained machine learning system is trained using a clustering algorithm, wherein the quantitative source estimation comprises an identification of a closest cluster, and wherein the source evaluation of the metal alloy sample comprises a score representative of a likelihood that the metal alloy sample belongs to the closest cluster.

9. The method of any one of claims 1-8, further comprising retraining (613) the trained machine learning system with a chemical composition quantification retraining corpus, wherein the chemical composition quantification retraining corpus comprises the chemical composition quantification.

10. The method of any one of claims 1-9, wherein the source evaluation of the metal sample comprises a plurality of scores representative of likelihoods that the metal alloy sample belongs to each of a plurality of previously identified clusters.

11. A system (100) for evaluating a metal alloy sample, the system comprising:
a portable hand-held X-ray fluorescence spectrometer configured to obtain spectroscopic data when applied to the metal alloy sample;
a non-transitory computer readable medium comprising instructions; and
at least one electronic processor (108) that executes the instructions to perform operations comprising:
obtaining (702) a chemical composition quantification (300, 400) for the metal alloy sample, wherein the chemical composition quantification is obtained from the spectroscopic data;
passing (704), automatically, the chemical composition quantification to a trained machine learning system (110, 605), from which a quantitative source estimation is obtained, wherein the trained machine learning system is trained with a chemical composition quantification training corpus, wherein the chemical composition quantification training corpus comprises a plurality of training chemical composition quantifications characterizing training metal alloy samples that originate from a plurality of metal alloy sources, thereby characterizing a metal alloy source fingerprint for each of the one or more metal alloy sources, the metal alloy source fingerprint representing an inherent chemical fingerprint; and
providing (706) a source evaluation (130) of the metal alloy sample, wherein the source evaluation of the metal alloy sample is based on the quantitative source estimation, and wherein the source evaluation of the metal alloy sample comprises a particular metal alloy source.

12. The system of claim 11, wherein:
the portable hand-held X-ray fluorescence spectrometer comprises the trained machine learning system; and/or
the portable hand-held X-ray fluorescence spectrometer comprises an integrated display (105), and wherein the providing comprises providing on the display.

13. The system of claim 11 or 12, wherein the trained machine learning system is communicatively coupled to the portable hand-held X-ray fluorescence spectrometer, and wherein the passing comprises automatically wirelessly transmitting.

14. The system of any one of claims 11-13, wherein the source evaluation of the metal alloy sample comprises:
one of a binary value, wherein the binary value corresponds to whether or not the metal alloy sample originated from the particular metal alloy source and was produced by a metal alloying process certified for the particular metal alloy source; and/or
a score, wherein the score characterizes a likelihood of whether the metal alloy sample originated from the particular metal alloy source and was produced by a metal alloying process certified for the particular metal alloy source; and/or
a plurality of scores representative of likelihoods that the metal alloy sample belongs to each of a plurality of previously identified clusters.

15. The system of any one of claims 11-14, wherein:
the quantitative source estimation matches the metal alloy source fingerprint that represents a particular metal alloying process of the particular metal alloy source; and/or
the trained machine learning system is trained using a clustering algorithm, wherein the quantitative source estimation comprises an identification of a closest cluster, and wherein the source evaluation of the metal alloy sample comprises a score representative of a likelihood that the metal alloy sample belongs to the closest cluster; and/or
the operations further comprise retraining (613) the trained machine learning system with a chemical composition quantification retraining corpus, wherein the chemical composition quantification retraining corpus comprises the chemical composition quantification.
